# FASCICULE DE BREVET EUROPEEN

(11) **EP 3 923 830 B1**
(45) Date de publication et mention de la délivrance du brevet: **03.09.2025**
(21) Numéro de dépôt: 20704534.5
(22) Date de dépôt: 13.02.2020
(51) Int. Cl.: A61B 17/17, A61B 17/88, A61B 17/16, A61B 90/00, A61B 17/70, A61B 34/30, A61B 17/68, A61B 17/00

(54) **SYSTEME POUR LE TRAITEMENT CHIRURGICAL DU RACHIS**
SYSTEM ZUR CHIRURGISCHEN BEHANDLUNG DER WIRBELSÄULE
SYSTEM FOR SURGICAL TREATMENT OF THE SPINE

(30) Priorité: 14.02.2019 FR 1901513
(43) Date de publication de la demande: 22.12.2021
(73) Titulaire: S.M.A.I.O, 69800 Saint-Priest (FR)
(72) Inventeur: BUTTIN, Romain, 69800 SAINT PRIEST (FR); DAUNE, Gautier, 69500 BRON (FR); ROUSSOULY, Pierre, 69450 SAINT CYR AU MONT D'OR (FR)
(74) Mandataire: Lavoix
(86) Numéro de dépôt international: PCT/EP2020/053728
(87) Numéro de publication internationale: WO 2020/165327

(56) Documents cités:
- EP-A1- 2 849 652
- EP-A2- 1 937 160
- WO-A1-2012/068641
- WO-A1-2016/019035
- US-A- 3 346 894
- US-A- 5 573 537
- US-A1- 2008 262 526
- US-A1- 2013 331 840
- US-A1- 2018 199 951
- US-B1- 6 443 956
- US-B1- 6 540 747

## Description

La présente invention concerne un système de traitement chirurgical du rachis.

Le traitement chirurgical de la colonne vertébrale de patients humains peut conduire à poser des implants rachidiens. Ainsi, pour réaliser par exemple une arthrodèse d'un segment de plusieurs vertèbres, des implants sont rapportés sur ces dernières de manière à les fixer entre elles. Ces implants incluent très souvent des vis dites pédiculaires, car à placer dans les pédicules des vertèbres. Les gestes chirurgicaux nécessaires à la mise en place de ces vis pédiculaires sont délicats à réaliser du fait des faibles dimensions des structures osseuses où placer les vis, de l'absence de visibilité et de la criticité des structures anatomiques avoisinantes, telles que la moelle épinière, la veine cave, l'aorte, etc. De plus, en cas de mauvais positionnement des vis pédiculaires, l'implant rachidien risque de casser ou de se desceller.

En pratique, ces gestes chirurgicaux sont actuellement réalisés par des chirurgiens orthopédistes et neuro-orthopédistes qui, après avoir dégagé un accès postérieur aux vertèbres, utilisent sur ces dernières des outils ad hoc, notamment des outils de perçage osseux et des outils de vissage. Pour orienter leurs gestes, les chirurgiens peuvent travailler « à main levée » en utilisant soit des repères anatomiques, soit des visées radiographiques fournies par un imageur de bloc tel qu'un amplificateur de brillance, soit un système informatique de navigation peropératoire. Les chirurgiens peuvent également être assistés par des robots chirurgicaux qui positionnent par rapport au rachis du patient à opérer un guide dans lequel ou sur lequel le chirurgien introduit ou place un outil que le chirurgien manipule lors de l'application de cet outil sur le rachis : ce guide de positionnement est par exemple un guide de perçage, qui est positionné par le robot avec précision en fonction de données peropératoires et qui est pourvu d'un trou dans lequel le chirurgien introduit un outil de perçage, étant remarqué que ce n'est pas le robot lui-même qui applique l'outil de perçage, comme proposé dans US 2008/199951.

Ces diverses techniques opératoires permettent de minimiser les risques de perforer de manière traversante la vertèbre et donc d'endommager des structures anatomiques environnantes, en particulier des structures vitales telles que la moelle épinière, la veine cave, l'aorte, etc... Leur résultat reste toutefois tributaire, au moins en partie, du geste du chirurgien et donc de l'expertise et du niveau de compétence de ce dernier. De plus, le positionnement finalement obtenu pour la vis pédiculaire par rapport à la vertèbre n'est pas totalement maitrisé car on constate que, lors du vissage, la direction de progression de la vis dans la vertèbre peut s'écarter légèrement de la trajectoire de placement souhaitée.

US 5 573 537 a proposé une mèche chirurgicale spéciale pour le rachis. Cette mèche comprend successivement une partie terminale distale, une partie intermédiaire et une partie terminale proximale. La partie terminale distale, qui est qualifiée d'élément de sondage dans US 5 573 537, peut présenter une forme globalement cylindrique, flanquée de deux biseaux opposés : cette partie terminale distale ne permet pas de percer l'os cortical d'une vertèbre, mais est prévue pour, après qu'une ouverture ait été faite au travers de l'os cortical de la vertèbre, être introduite dans cette ouverture puis s'enfoncer dans l'os spongieux du pédicule de la vertèbre en y formant un avant-trou. La partie intermédiaire de la mèche présente, elle aussi, une forme globalement cylindrique et est pourvue d'éléments coupants permettant, lorsque la mèche est entraînée en rotation sur elle-même, de creuser dans le pédicule un trou arrière d'ancrage. Ce trou arrière d'ancrage est cylindrique de manière que ce trou arrière d'ancrage et l'avant-trou précité forment conjointement un trou étagé. La partie terminale proximale présente une forme cylindrique lisse, qui prolonge la partie intermédiaire et qui, à l'opposé de cette dernière, présente un évasement tronconique en formant un collier à pattes destiné à connecter mécaniquement la mèche à un outil d'entraînement en rotation : la surface extérieure lisse de cette partie terminale proximale est munie de lignes circonférentielles constituant des indices de pénétration. Lors du creusement du trou arrière d'ancrage, la partie terminale distale de la mèche assure un certain guidage de la mèche en coopérant avec la matière spongieuse au niveau de l'avant-trou. En revanche, la forme étagée du trou final obtenu n'améliore pas le placement ultérieur d'une vis pédiculaire.

EP 1 937 160 a proposé une mèche pour le rachis, qui est fonctionnellement similaire à celle de US 5 573 537.

US 6 443 956 a proposé une mèche vertébrale comprenant une tige coupante s'étendant depuis une extrémité distale jusqu'à une tête proximale. La tige coupante est pourvue d'une flute à bord tranchant, qui s'étend sur toute la longueur de la tige coupante. L'extrémité distale présente des bords émoussés qui sont spécifiquement conçus pour être incapables de couper en travers l'os cortical, de sorte qu'il est nécessaire de pratiquer une ouverture dans l'os dur cortical d'un pédicule préalablement à l'introduction, dans cette ouverture, de l'extrémité distale émoussée de la mèche.

Plutôt qu'une mèche, US2013/331840 a envisagé un instrument d'accès osseux pour la chirurgie du rachis. Cet instrument comporte une tige incluant deux parties de tige successives, respectivement proximale et distale. La partie de tige distale présente une extrémité distale pointue et une surface latérale cylindrique qui est lisse. La partie de tige proximale, qui est bien plus longue que la partie de tige distale, présente une surface latérale cylindrique, qui est potentiellement pourvue de cannelures à arête coupante, et qui, à son extrémité proximale, est équipée d'une poignée de préhension.

US2008/262526A1 concerne une mèche de neurostimulation.

Le but de la présente invention est de proposer un système de traitement chirurgical du rachis, qui améliore le placement de vis pédiculaires dans les vertèbres.

A cet effet, l'invention a pour objet un système de traitement chirurgical du rachis, tel que défini à la revendication 1.

Une des idées à la base de l'invention est de préparer la trajectoire de placement d'une vis pédiculaire en réalisant, préalablement à l'application de cette vis sur une vertèbre d'un patient, un trou dans le pédicule de la vertèbre, ce trou étant percé par une mèche spécifique qui donne à la partie terminale proximale du trou une forme d'entonnoir : de cette façon, lorsque la vis pédiculaire est présentée à l'entrée de ce trou, l'extrémité distale de la vis pédiculaire peut coopérer par contact avec l'entonnoir de manière à centrer la vis pédiculaire dans le trou et donc à l'aligner avec l'axe du trou, cet axe ayant été positionné avec précision sur la vertèbre par la mèche lors du perçage du trou. Ainsi, lorsque la vis pédiculaire est appliquée sur la vertèbre de manière décalée par rapport à la trajectoire qu'avait suivie la mèche pour percer le trou, la vis pédiculaire est naturellement ramenée dans l'axe du trou. La forme d'entonnoir résulte de la présence d'une fraise, par exemple conique, qui est prévue à l'extrémité proximale d'un foret cylindrique reliant la fraise à l'extrémité distale de la mèche. Ce foret, qui donne une forme cylindrique à la partie terminale distale du trou, présente un diamètre inférieur au diamètre nominal de la tige filetée de la vis pédiculaire, afin que cette tige filetée soit guidée coaxialement dans la partie terminale distale du trou lors du vissage. Grâce à l'invention, le placement de la vis pédiculaire est ainsi particulièrement précis.

De plus, ce placement peut avantageusement être contrôlé de manière peropératoire avant de visser la vis pédiculaire, moyennant l'utilisation d'un organe ad hoc, tel qu'une sonde métallique, qui est introduite dans le trou et qui permet au chirurgien de facilement contrôler matériellement le positionnement du trou au sein de la vertèbre, par exemple par palpation directe de cet organe ou bien par radiographie peropératoire.

Le système selon l'invention peut être utilisé manuellement par le chirurgien, c'est-à-dire que la mèche et la vis pédiculaire sont alors entraînées en rotation par des dispositifs motorisés ad hoc, tenus à la main par le chirurgien. Cela dit, l'invention trouve une application particulièrement avantageuse pour le cas où les dispositifs motorisés d'entraînement de la mèche et de la vis pédiculaire sont « tenus » par un robot chirurgical, autrement dit déplacés dans l'espace par ce robot, étant souligné que c'est le robot qui réalise alors lui-même l'acte chirurgical de perçage et de vissage dans les vertèbres, et ce avec précision et répétabilité. Dans ce cas, pour améliorer encore davantage les performances du système de traitement chirurgical correspondant, en évitant un écart de placement dans la vertèbre par rapport au positionnement qui est demandé au robot par le chirurgien, le système peut avantageusement être prévu pour ajuster le déplacement des dispositifs motorisés par le robot et donc commander en position la mèche lors du perçage du trou et commander en position la vis pédiculaire lors du vissage dans le trou, en tenant compte des efforts exercés sur la vertèbre du patient par la mèche lors du perçage et par la vis pédiculaire lors du vissage, comme expliqué plus en détail par la suite.

Des caractéristiques additionnelles avantageuses du système de traitement chirurgical conforme à l'invention sont spécifiées aux autres revendications.

On notera que les considérations techniques relatives à la commande en déplacement par le robot, en fonction de la mesure des efforts exercés lors du perçage ou du vissage, peuvent être mises en œuvre indépendamment des spécificités de la mèche et de la vis pédiculaire.

L'invention sera mieux comprise à la lecture de la description qui va suivre, donnée uniquement à titre d'exemple et faite en se référant aux dessins sur lesquels :
[Fig 1] la figure 1 est une vue en élévation d'une mèche et d'une vis pédiculaire qui appartiennent à un système de traitement chirurgical conforme à l'invention ;
[Fig 2] la figure 2 est un schéma de la mèche de la figure 1, associée à d'autres éléments du système de traitement conforme à l'invention et montrée en cours d'utilisation sur une vertèbre humaine, observée en coupe transversale ; et
[Fig 3] la figure 3 et
[Fig 4] la figure 4 sont des vues similaires à la figure 2, illustrant l'application sur la vertèbre de la vis pédiculaire de la figure 1 par le système de traitement chirurgical.

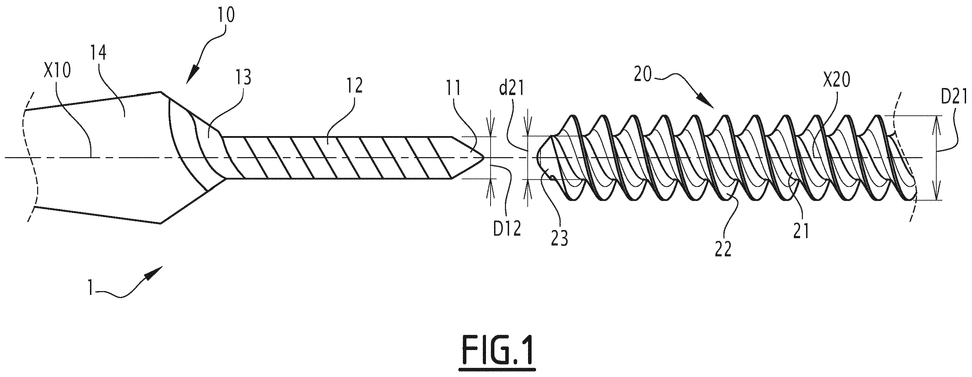

Sur les figures 1 à 4 est représenté un système 1 permettant de réaliser un traitement chirurgical du rachis humain.

Ce système 1 comprend une mèche 10 et une vis pédiculaire 20, montrées seules sur la figure 1.

La mèche 10 présente une forme globalement allongée et définit un axe de mèche X10 proximo-distal, le long et autour duquel s'étend en longueur la mèche 10. La mèche 10 inclut, successivement suivant l'axe de mèche X10, une extrémité distale 11, un foret 12, une fraise 13 et une queue d'extrémité proximale 14.

Le foret 12 présente une forme cylindrique, centrée sur l'axe de mèche X10. Lorsque la mèche 10 est entraînée en rotation sur elle-même autour de l'axe de mèche X10, le foret 12 est à même de percer un trou cylindrique dans une matière osseuse, le diamètre du trou cylindrique obtenu correspondant sensiblement au diamètre D12 du foret 12. A cet effet, le foret 12 est par exemple pourvu de rainures hélicoïdales à arête tranchante, qui s'enroulent autour de l'axe de mèche X10 le long du foret 12. Les spécificités du foret 12, permettant l'entame et l'évacuation de la matière osseuse pour y réaliser le trou cylindrique précité, ne sont pas limitatives de l'invention.

La fraise 13 présente, quant à elle, une forme s'élargissant, par rapport à l'axe de mèche X10, de manière progressive en direction proximale depuis le foret 12. La fraise 13 présente ainsi, à son extrémité distale de jonction avec le foret 12, un diamètre minimal, correspondant au diamètre D12 du foret, tandis que, à son extrémité proximale de jonction avec la queue d'extrémité proximale 14, la fraise 13 présente un diamètre maximal, supérieur au diamètre minimal précité. Entre ses extrémités distale et proximale, la fraise 13 présente un diamètre croissant, en particulier continument croissant, suivant l'axe de mèche X10. Suivant une forme de réalisation pratique qui est mise en œuvre dans l'exemple considéré sur les figures, la fraise 13 présente ainsi une forme conique, qui est centrée sur l'axe de mèche X10 et qui diverge en direction proximale depuis le foret 12. Quelles que soient les spécificités géométriques de la fraise 13, cette fraise permet, lorsque la mèche 10 est entraînée en rotation sur elle-même autour de l'axe de mèche X10, de réaliser dans une matière osseuse une cavité en forme d'entonnoir s'évasant progressivement en direction proximale depuis l'extrémité distale de cette cavité. A cet effet, la fraise 13 est par exemple pourvue de reliefs externes à arête coupante, qui entaillent mécaniquement la matière osseuse pour y réaliser la cavité en forme d'entonnoir : les spécificités structurelles de la fraise 13, permettant d'entailler la matière osseuse pour y réaliser la cavité en forme d'entonnoir, ne sont pas limitatives de l'invention.

Pour permettre de planter la mèche 10 dans une matière osseuse à percer, l'extrémité distale 11 présente une forme pointue, typiquement conique, qui est centrée sur l'axe de mèche X10.

La vis pédiculaire 20, qui est destinée à être placée dans le pédicule d'une vertèbre humaine, définit un axe de vis X20 proximo-distal, le long et autour duquel la vis pédiculaire 20 s'étend en longueur. La vis pédiculaire 20 inclut, en partie distale, une tige filetée 21 et, en partie proximale, une tête : sur la figure 1, seule la tige filetée 21 est représentée, étant remarqué que la tête de la vis pédiculaire 20 n'est pas limitative de l'invention du moment que, de manière connue en soi, cette tête est à même d'être engagée mécaniquement pour entraîner en rotation sur elle-même la vis pédiculaire 20 autour de l'axe de vis X20.

La tige filetée 21 présente une forme cylindrique, qui est centrée sur l'axe de vis X20 et dont le diamètre nominal D21 est, de manière usuelle, défini par l'arête externe d'un filetage 22 s'enroulant autour de l'axe de vis X20 le long de la tige filetée 21. Ce diamètre nominal D21 est supérieur au diamètre D12 du foret 12, comme bien visible sur la figure1. Suivant ce qui est illustré sur les figures, le diamètre D12 du foret 12 est sensiblement égal au diamètre d21 de la tige filetée 21, mesuré à la base du filetage 22.

La tige filetée 21 inclut une extrémité distale 23 qui, comme montré sur la figure 1, constitue l'extrémité distale de la vis pédiculaire 20. Suivant une forme de réalisation qui est avantageuse pour des raisons qui apparaîtront plus loin, cette extrémité distale 23 présente une forme sensiblement conique, qui est centrée sur l'axe de vis X20 et qui diverge en direction proximale.

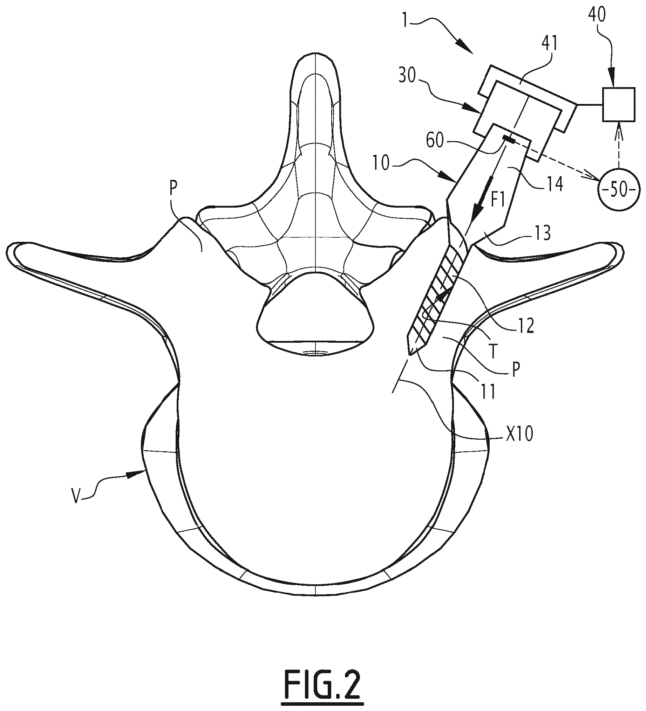

Le système de traitement chirurgical 1 comporte d'autres éléments qui vont être présentés ci-après dans le cadre de la description, en regard des figures 2 à 4, de l'utilisation de la mèche 10 et de la vis pédiculaire 20 successivement appliquées à une vertèbre V du rachis d'un patient.

L'utilisation de la mèche 10 et de la vis pédiculaire 20 est mise en œuvre dans le cadre d'une intervention chirurgicale, au cours de laquelle on accède d'abord à la vertèbre V par voie postérieure.

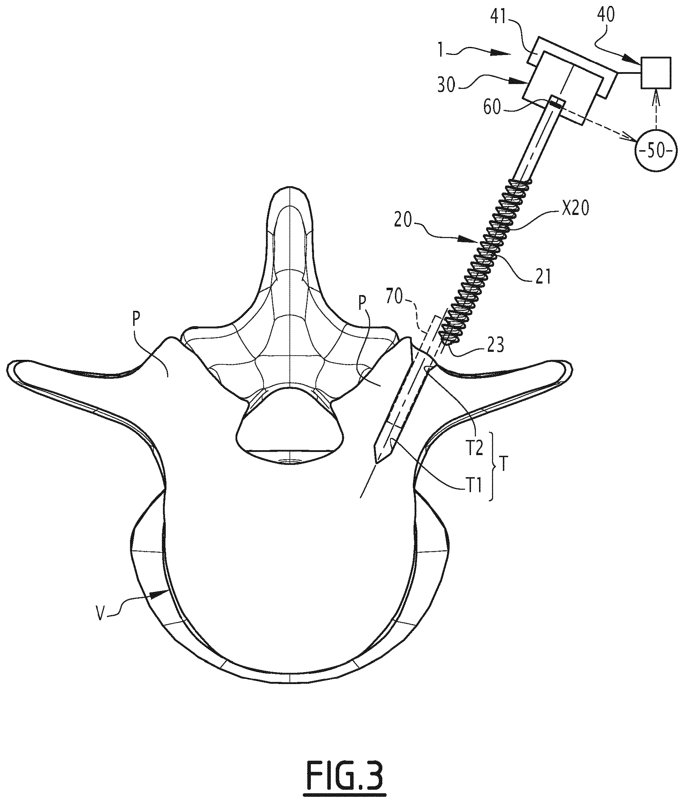

Puis, la mèche 10 est utilisée lors d'une étape de perçage au cours de laquelle la mèche 10 perce un trou T dans un des pédicules P de la vertèbre V. Pour ce faire, la mèche 10 est d'abord déplacée dans l'espace pour être rapprochée de la vertèbre, jusqu'à être appliquée sur la vertèbre de manière à planter dans cette dernière l'extrémité distale 11 de la mèche 10, la forme pointue de l'extrémité distale 11 permettant une application franche et non glissante de la mèche sur la vertèbre. Puis la mèche 10 est alors entraînée en rotation autour de l'axe de mèche X10, tout en étant déplacée dans l'espace de manière à progresser dans le pédicule P de la vertèbre V, cette progression de la mèche 10 étant opérée suivant une direction de déplacement qui est indiquée par une flèche F1 sur la figure 2 et qui s'étend parallèlement à l'axe de mèche X10. Du fait de son entrainement en rotation et son déplacement, la mèche 10 perce progressivement le pédicule P, et ce jusqu'à ce que la fraise 13 atteigne et progresse dans la vertèbre V, étant noté que la queue d'extrémité proximale 14 reste toujours à l'extérieur de la vertèbre V. Le trou T ainsi percé par la mèche 10, qui est mieux visible sur la figure 3, inclut une partie terminale distale T1, présentant une forme cylindrique qui lui a été donnée par le foret 12, et une partie terminale proximale T2, présentant une forme d'entonnoir s'évasant progressivement depuis la partie terminale distale T1, cette forme d'entonnoir lui ayant été donnée par la fraise 13 en fin de course de la mèche 10.

En pratique, le diamètre D12 du foret 12 est suffisamment petit pour minimiser le risque d'endommager les parois latérales du pédicule P lors de l'étape de perçage, le pédicule s'apparentant à un tube osseux ayant un diamètre variable de 5 à 12 mm. De plus, le foret 12 est prévu relativement court, dans le sens où, à l'issue de l'étape de perçage, l'extrémité distale 11 de la mèche 10 atteint le corps vertébral de la vertèbre V, sans toutefois y être profondément enfoncé. De cette façon, à l'issue de l'étape de perçage, la mèche 10 atteint le corps vertébral de la vertèbre V, sans prendre le risque de dépasser les limites osseuses antérieures de la vertèbre, qui protègent des structures anatomiques nobles, notamment vasculaires, en avant de la vertèbre.

Afin d'entraîner la mèche 10 en rotation lors de l'étape de perçage, le système de traitement chirurgical 1 comporte un dispositif motorisé 30. Les spécificités de ce dispositif motorisé 30 aux fins de l'entraînement de la mèche 10 ne sont pas limitatives : à titre d'exemple, le dispositif motorisé 30 inclut un moteur électrique dont l'arbre de sortie est couplé en rotation autour de l'axe de mèche X10 à la queue d'extrémité proximale 14, comme illustré schématiquement sur la figure 2. De plus, afin de déplacer la mèche 10 dans l'espace, en particulier suivant la direction de déplacement F1 lors de la progression de la mèche 10 dans le pédicule P, le système de traitement chirurgical 1 comporte un robot 40 qui n'est représenté que de manière schématique sur la figure 2. Ce robot 40 consiste par exemple en un bras robotisé se terminant par un organe effecteur 41 à même d'être solidarisé fixement au dispositif motorisé 30. Quelle que soit la forme de réalisation du robot 40, ce robot 40 est adapté pour déplacer dans l'espace le dispositif motorisé 30.

Lors de l'intervention chirurgicale, le déplacement du dispositif motorisé 30 et, par-là, de la mèche 10, par le robot 40 est commandé par une unité électronique 50 recevant ses instructions d'un chirurgien. Bien entendu, les déplacements opérés par le robot 40 sont repérés dans l'espace, en étant réalisés par rapport à un référentiel qui est connu de l'unité électronique 50 et dans lequel est positionnée la vertèbre V. Ainsi, après que le chirurgien ait décidé de l'emplacement qu'il souhaite donner au trou T dans le pédicule P de la vertèbre V, notamment sur la base de données préopératoire et/ou peropératoire relatives à ce pédicule P, le chirurgien donne des instructions correspondantes à l'unité électronique 50 qui commande alors le robot 40 pour déplacer par rapport à la vertèbre V le dispositif motorisé 30 et, par-là, la mèche 10 entraînée en rotation par ce dispositif, de manière que la mèche soit appliquée sur cette dernière et progresse dans le pédicule P suivant la direction de déplacement F1.

Suivant une disposition optionnelle avantageuse, le déplacement du dispositif motorisé 30 par le robot 40 peut être corrigé au cours de l'étape de perçage lorsqu'il est constaté que la direction de déplacement F1 dévie de l'axe du trou T en cours de réalisation, autrement dit lorsque la direction de déplacement F1 tend à perdre son parallélisme avec l'axe de mèche X10. A cet effet, l'unité électronique 50 est adaptée, en particulier programmée, pour commander le déplacement opéré par le robot 40 en fonction d'une ou des composantes spatiales des efforts qu'exerce la mèche 10 sur la vertèbre V lors du perçage du trou T. En pratique, cette ou ces composantes de ces efforts sont mesurées par un capteur d'effort 60, qui est intégré au dispositif motorisé 30 et qui est par exemple adapté pour fournir un signal électrique représentatif de la déformation de la mèche 10 lors du perçage du trou T. Moyennant un traitement prédéterminé de la ou des composantes d'effort mesurées par le capteur d'effort 60, l'unité électronique 50 assure une commande en position du robot 40, qui est gardée en effort et qui est prévue pour maintenir la direction de déplacement F1 alignée avec l'axe de mèche X10 et donc avec l'axe du trou T en cours de réalisation, lors du perçage de ce trou par la mèche 10. On garde ainsi les bénéfices d'une commande en effort, tout en limitant, voire en éliminant la problématique d'éventuelles instabilités du perçage notamment liées à de légers changements des orientations de la mèche 10, dus par exemple à des jeux, et/ou liées à des variations des caractéristiques de l'environnement de perçage, ces variations étant par exemple dues à de faibles mouvements du patient ou à une inhomogénéité de la matière osseuse percée : la modification des efforts exercés par la mèche 10 sur la vertèbre V lors du perçage du trou T est en effet révélateur de telles instabilités.

Suivant une mise en œuvre préférentielle de cette méthode de commande en position, opérée par l'unité électronique 50, cette dernière est configurée, notamment programmée, pour analyser la ou les composantes d'effort mesurées par le capteur d'effort 60, puis pour commander le robot 40 à partir du résultat de cette analyse. Plusieurs possibilités peuvent être envisagées pour la teneur de l'analyse mise en œuvre par l'unité électronique 50 : cette dernière est par exemple configurée pour comparer la ou chacune des composantes d'effort à une valeur prédéterminée, ou bien pour suivre dans le temps cette ou ces composantes d'effort pour en détecter une dérive temporelle. L'unité électronique 50 peut ainsi être configurée pour commander le robot 40 de manière à garder la ou chacune des composantes d'effort constante, à une marge prédéterminée près de l'ordre de quelques pourcents.

Une fois que le trou T est réalisé à l'issue de l'étape de perçage, le traitement chirurgical de la vertèbre V se poursuit par une étape de vissage au cours de laquelle la vis pédiculaire 20 est vissée dans ce trou T. Ceci étant, le traitement chirurgical peut inclure une étape optionnelle intermédiaire entre l'étape de perçage et l'étape de vissage, cette étape intermédiaire étant destinée à contrôler le positionnement du trou T dans la vertèbre V. Pour ce faire, le système de traitement 1 comporte un organe de contrôle peropératoire 70, représenté uniquement de manière schématique en pointillés sur la figure 3. Cet organe de contrôle peropératoire 70 comporte, voire consiste en une sonde, telle qu'une tige, à même d'être introduite dans le trou T, comme illustré à la figure 3. Quelle que soit sa forme de réalisation, l'organe de contrôle peropératoire 70 permet au chirurgien de contrôler que le trou T, ayant été percé par la mèche 10 dans la vertèbre V, est positionné dans cette dernière de manière convenable, en particulier conformément à ce que le chirurgien avait prévu par les instructions de commande qu'il avait données à l'unité électronique 50. Le contrôle effectué par le chirurgien peut être réalisé directement par palpation de l'organe de contrôle peropératoire 70, plus précisément de la partie de ce dernier qui émerge du trou T. Ce contrôle peut aussi être réalisé par radiographie peropératoire dès lors que l'organe de contrôle peropératoire 70 est radio-opaque, par exemple métallique. Le chirurgien peut ainsi s'assurer facilement que le vissage à venir de la vis pédiculaire 20 peut être enchaîné sans danger pour le patient.

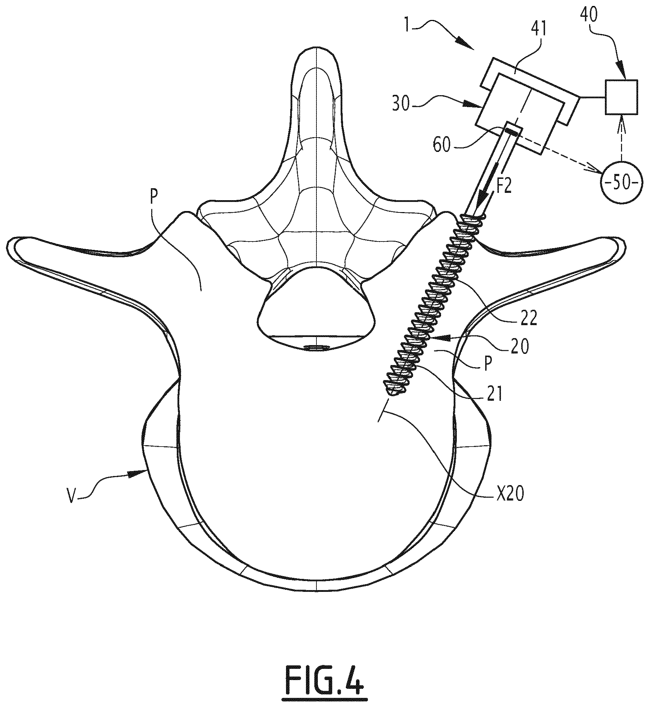

Au cours de l'étape de vissage, la vis pédiculaire 20 est entraînée en rotation autour de l'axe de vis X20 et est déplacée par rapport à la vertèbre V de façon à être introduite et à progresser dans le trou T, comme illustré par les figures 3 et 4. A cette fin, le dispositif motorisé 30 et le robot 40 sont utilisés sensiblement de la même façon que lors de l'étape de perçage, en étant cette fois-ci appliqués à la vis pédiculaire 20 : en particulier, le dispositif motorisé 30 est connecté à la tête de la vis pédiculaire 20 aux fins d'entraînement en rotation de cette dernière autour de l'axe de vis X20, et le déplacement de la tige filetée 21 dans le trou T, opéré par le robot 40 est réalisé selon une direction qui est notée F2 sur la figure 4 et qui s'étend parallèlement à l'axe de vis X20. Bien entendu, le dispositif motorisé et/ou le robot utilisés lors de l'étape de vissage peuvent ne pas être exactement les mêmes que le dispositif motorisé 30 et/ou le robot 40 utilisés lors de l'étape de perçage, mais sont fonctionnement similaires à ces derniers et sont donc représentés et référencés sur les figures 3 et 4 de la même façon que sur la figure 2.

La figure 3 illustre le moment où la vis pédiculaire 20 se présente à l'entrée du trou T, avant de s'engager dans la profondeur de ce dernier. En principe, la vis pédiculaire 20 est présentée par le robot 40 à l'entrée du trou T de manière que l'axe de vis X20 soit centré sur ce trou. Toutefois, pour diverses raisons, notamment liées à des jeux ou des imprécisions de fabrication et/ou liées à de faibles mouvements du patient, l'axe de vis X20 peut ne pas être rigoureusement aligné sur l'axe du trou T, comme illustré sur la figure 3. Dès lors que l'extrémité distale 23 de la tige filetée 21 commence d'être introduite dans le trou T, elle entre en contact avec la partie terminale proximale T2 du trou T, en forme d'entonnoir, de manière que cette partie terminale proximale T2 ramène, par effet de rampe, la vis pédiculaire 20 dans l'axe du trou T. Autrement dit, la coopération de contact entre la forme en entonnoir de la partie terminale proximale T2 du trou T et l'extrémité distale 23 de la tige filetée 21 produit un effet de centrage de la vis pédiculaire 20 dans le trou T. Cet effet de centrage est avantageusement renforcé grâce à la forme conique de l'extrémité distale 23. Dans tous les cas, lorsque l'extrémité distale 23 de la tige filetée 21 atteint et commence de progresser dans la partie terminale distale T1 du trou T, la vis pédiculaire 20 est coaxiale au trou T.

La vis pédiculaire 20 progresse ensuite à l'intérieur du trou T, son filetage 22 venant mettre en prise la paroi de la partie terminale distale T1 du trou T au fur et à mesure du vissage de la vis pédiculaire 20. La tige filetée 21 s'enfonce ainsi coaxialement dans la partie terminale distale T1 du trou T, et ce d'autant plus précisément que le diamètre de cette partie terminale distale T1 est sensiblement égal au diamètre d21 de la tige filetée, mesuré à la base du filetage 22. Le cas échéant, la vis pédiculaire 20 pénètre dans la vertèbre V au-delà de l'extrémité distale de la partie terminale distale T1 du trou T, en s'enfonçant sans difficulté dans la matière osseuse spongieuse du corps vertébral de la vertèbre V.

Suivant des considérations similaires à celles développées plus haut en lien avec la méthode de commande en position, gardée en effort, que met en œuvre l'unité électronique 50 lors de l'étape de perçage, le déplacement par le robot 40 du dispositif motorisé 30 et, par-là, de la vis pédiculaire 20 entraînée en rotation par ce dernier est avantageusement commandé en fonction d'une ou des composantes des efforts exercés par la vis pédiculaire 20 sur la vertèbre V lors du vissage dans le trou T. Ainsi, moyennant un traitement de la ou des composantes de ces efforts, mesurées par le capteur 60, l'unité électronique 50 assure une commande en position du robot 40, qui est gardée en effort et qui est prévue pour maintenir la direction de déplacement F2 alignée avec l'axe de vis X20 et donc avec l'axe du trou T, lors du vissage de la vis pédiculaire 20 dans le trou. En pratique, les explications données plus haut en lien avec l'étape de perçage s'appliquent mutatis mutandis à l'étape de vissage.

Par ailleurs, divers aménagements et variantes au système, décrit jusqu'ici, sont envisageables. A titre d'exemple, plutôt que la mèche 10 et/ou la vis pédiculaire 20 soient entraînées en rotation et déplacées dans l'espace par le dispositif motorisé 30 et le robot 40, cette mèche et/ou cette vis pédiculaire peuvent être sollicitées manuellement par le chirurgien.

## Revendications

1. Système (1) de traitement chirurgical du rachis, comportant :
- une mèche (10), qui s'étend suivant un axe de mèche (X10) proximo-distal et qui est adaptée pour percer un trou (T) dans le pédicule (P) d'une vertèbre (V) d'un patient lorsque la mèche est entraînée en rotation sur elle-même autour de l'axe de mèche, laquelle mèche inclut successivement suivant l'axe de mèche :
- une extrémité distale (11),
- un foret cylindrique (12), qui est centré sur l'axe de mèche (X10) et qui est prévu pour percer le trou (T) en entamant et évacuant de la matière osseuse du pédicule (P) de la vertèbre (V) de manière à donner une forme de cylindre à une partie terminale distale (T1) du trou, et
- une fraise (13) adaptée pour entailler la matière osseuse du pédicule de la vertèbre de manière à donner à une partie terminale proximale (T2) du trou (T) une forme d'entonnoir s'évasant progressivement depuis la partie terminale distale (T1) du trou (T), et
- une vis pédiculaire (20), qui s'étend suivant un axe de vis (X20) proximo-distal,
**caractérisé en ce que** :
- l'extrémité distale (11) est pointue et centrée sur l'axe de mèche (X1) pour pouvoir être plantée dans une matière osseuse à percer du pédicule (P) de la vertèbre (V),
- la vis pédiculaire (20) est adaptée pour, après que la mèche (10) ait percé le trou (T) dans le pédicule (P) de la vertèbre (V) et ait été retirée de ce trou, être vissée dans le trou en y étant entraînée en rotation sur elle-même autour de l'axe de vis, laquelle vis pédiculaire inclut une tige filetée (21), qui comprend une extrémité distale (23) constituant une extrémité distale de la vis pédiculaire (20), et qui présente une forme cylindrique, centrée sur l'axe de vis (X20) et dont le diamètre nominal (D21) est supérieur au diamètre (D12) du foret (12), le diamètre (D12) du foret (12) étant sensiblement égal au diamètre (d21) de la tige filetée (21), mesuré à la base du filetage (22) de la tige filetée.

2. Système suivant la revendication 1, dans lequel la fraise (13) présente une forme conique, qui est centrée sur l'axe de mèche (X10) et qui diverge en direction proximale depuis le foret (12).

3. Système suivant l'une des revendications 1 ou 2, dans lequel le foret (12) est pourvu de rainures hélicoïdales à arête tranchante, qui s'enroulent autour de l'axe de mèche (X10) le long du foret.

4. Système suivant l'une quelconque des revendications précédentes, dans lequel l'extrémité distale (23) de la tige filetée (21) présente une forme sensiblement conique, qui est centrée sur l'axe de vis (X20) et qui diverge en direction proximale.

5. Système suivant l'une quelconque des revendications précédentes, dans lequel le système (1) comporte en outre un organe de contrôle peropératoire (70), qui est adapté pour, après que la mèche (10) ait percé le trou (T) dans le pédicule (P) de la vertèbre (V) et ait été retirée de ce trou et avant que la vis pédiculaire (20) soit vissée dans le trou, être introduit dans le trou de manière à contrôler, notamment par palpation et/ou radiographie, le positionnement du trou dans la vertèbre.

6. Système suivant l'une quelconque des revendications précédentes, dans lequel le système (1) comporte en outre :
- un dispositif motorisé (30) adapté pour entraîner en rotation la mèche (10) autour de l'axe de mèche (X10) et/ou pour entraîner en rotation la vis pédiculaire (20) autour de l'axe de vis (X20), et
- un robot (40) adapté pour déplacer dans l'espace le dispositif motorisé (30).

7. Système suivant la revendication 6, dans lequel le dispositif motorisé (30) est équipé d'un capteur d'effort (60) configuré pour mesurer au moins une composante des efforts exercés sur la vertèbre (V) par la mèche (10) lors du perçage du trou (T) et/ou par la vis pédiculaire (20) lors du vissage dans le trou, et dans lequel le système (1) comporte en outre une unité électronique (50) adaptée pour commander le déplacement du dispositif motorisé (30) par le robot (40) en fonction de ladite au moins une composante mesurée par le capteur d'effort (60).

8. Système suivant la revendication 7, dans lequel l'unité électronique (50) est configurée, à la fois, pour analyser ladite au moins une composante mesurée par le capteur d'effort (60), notamment par comparaison de ladite au moins une composante à une valeur prédéterminée ou par suivi temporel de ladite au moins une composante, et pour commander le déplacement du dispositif motorisé (30) par le robot (40) à partir du résultat de l'analyse de ladite au moins une composante.

9. Système suivant l'une des revendications 7 ou 8, dans lequel l'unité électronique (50) est configurée pour commander le déplacement du dispositif motorisé (30) par le robot (40) de manière à garder sensiblement constante ladite au moins une composante mesurée par le capteur d'effort (60).

## Patentansprüche

1. System (1) zur chirurgischen Behandlung der Wirbelsäule, umfassend:
- einen Bohrer (10), der sich entlang einer proximo-distalen Bohrerachse (X10) erstreckt und angepasst ist, um ein Loch (T) in den Pedikel (P) eines Wirbels (V) eines Patienten zu bohren, wenn der Bohrer um die Bohrerachse um sich selbst gedreht wird, wobei der Bohrer nacheinander entlang der Bohrerachse Folgendes beinhaltet:
- ein distales Ende (11),
- einen zylindrischen Bohrer (12), der auf der Bohrerachse (X10) zentriert ist und zum Bohren des Lochs (T) durch Anschneiden und Entfernen von Knochenmaterial von dem Pedikel (P) des Wirbels (V) bereitgestellt ist, um einem distalen Endabschnitt (T1) des Lochs eine Zylinderform zu verleihen, und
- einen Fräser (13), der angepasst ist, um das Knochenmaterial des Wirbelstiels einzuschneiden, um einem proximaler Endabschnitt (T2) des Lochs (T) eine Trichterform zu verleihen, die sich allmählich von dem distalen Endabschnitt (T1) des Lochs (T) aus erweitert, und
- eine Pedikelschraube (20), die sich entlang einer proximo-distalen Schraubenachse (X20) erstreckt,
**dadurch gekennzeichnet, dass**:
- das distale Ende (11) spitz und auf die Bohrerachse (X1) zentriert ist, um in ein zu durchstechendes Knochenmaterial des Pedikels (P) des Wirbels (V) getrieben werden zu können,
- die Pedikelschraube (20) angepasst ist, um nachdem der Bohrer (10) das Loch (T) in den Pedikel (P) des Wirbels (V) gebohrt hat und aus diesem Loch herausgezogen wurde, in das Loch eingeschraubt zu werden, indem sie dort um sich selbst um die Schraubenachse gedreht wird, wobei die Pedikelschraube einen Gewindeschaft (21) beinhaltet, der ein distales Ende (23) aufweist, das ein distales Ende der Pedikelschraube (20) darstellt, und die eine zylindrische Form aufweist, die auf der Schraubenachse (X20) zentriert ist und deren Nenndurchmesser (D21) größer ist als der Durchmesser (D12) des Bohrers (12), wobei der Durchmesser (D12) des Bohrers (12) im Wesentlichen gleich wie der Durchmesser (d21) der Gewindestange (21), gemessen an der Basis des Gewindes (22) der Gewindestange, ist.

2. System nach Anspruch 1, wobei der Fräser (13) eine konische Form aufweist, die auf der Bohrerachse (X10) zentriert ist und proximal von dem Bohrer (12) divergiert.

3. System nach einem der Ansprüche 1 oder 2, wobei der Bohrer (12) mit schraubenförmigen, scharfkantigen Nuten versehen ist, die sich um die Bohrerachse (X10) entlang des Bohrers winden.

4. System nach einem der vorherigen Ansprüche, wobei das distale Ende (23) der Gewindestange (21) eine im Wesentlichen konische Form aufweist, die auf der Schraubenachse (X20) zentriert ist und in proximaler Richtung divergiert.

5. System nach einem der vorherigen Ansprüche, wobei das System (1) ferner ein intraoperatives Kontrollorgan (70) umfasst, das dazu geeignet ist, nachdem der Bohrer (10) das Loch (T) in den Pedikel (P) des Wirbels (V) gebohrt hat und aus diesem Loch herausgezogen wurde und bevor die Pedikelschraube (20) in das Loch geschraubt wird, in das Loch eingeführt zu werden, um insbesondere durch Abtasten und/oder Röntgen die Positionierung des Lochs in dem Wirbel zu kontrollieren.

6. System nach einem der vorherigen Ansprüche, wobei das System (1) ferner Folgendes umfasst:
- eine motorisierte Vorrichtung (30), die angepasst ist, um den Bohrer (10) um die Bohrerachse (X10) zu drehen und/oder die Pedikelschraube (20) um die Schraubenachse (X20) zu drehen, und
- einen Roboter (40), der angepasst ist, um die motorisierte Vorrichtung (30) im Raum zu bewegen.

7. System nach Anspruch 6, wobei die motorisierte Vorrichtung (30) mit einem Kraftsensor (60) ausgestattet ist, der konfiguriert ist, um mindestens eine Komponente der Kräfte zu messen, die durch den Bohrer (10) beim Bohren des Lochs (T) und/oder durch die Pedikelschraube (20) beim Einschrauben in das Loch auf den Wirbel (V) ausgeübt werden, und wobei das System (1) ferner eine elektronische Einheit (50) umfasst, die angepasst ist, um die Bewegung der motorisierten Vorrichtung (30) durch den Roboter (40) abhängig von der mindestens einen durch den Kraftsensor (60) gemessenen Komponente zu steuern.

8. System nach Anspruch 7, wobei die elektronische Einheit (50) konfiguriert ist, um sowohl die mindestens eine von dem Kraftsensor (60) gemessene Komponente zu analysieren, insbesondere durch Vergleich der mindestens einen Komponente mit einem vorbestimmten Wert oder durch zeitliche Verfolgung der mindestens einen Komponente, als auch die Bewegung der motorisierten Vorrichtung (30) durch den Roboter (40) anhand des Resultats der Analyse der mindestens einen Komponente zu steuern.

9. System nach einem der Ansprüche 7 oder 8, wobei die elektronische Einheit (50) konfiguriert ist, um die Bewegung der motorisierten Vorrichtung (30) durch den Roboter (40) zu steuern, sodass die mindestens eine von dem Kraftsensor (60) gemessene Komponente im Wesentlichen konstant gehalten wird.

## Claims

1. A system (1) for surgical treatment of the spine, comprising:
- a drill bit (10), that extends along a proximo-distal drill axis (X10) and that is suitable for drilling a hole (T) in the pedicle (P) of a vertebra (V) of a patient when the drill bit is rotated on itself about the drill axis, this drill bit including, successively, along the drill axis:
- a distal end (11),
- a cylindrical drill (12), centered on the drill axis (X10) and suitable for drilling the hole (T) by cutting into and removing bone material from the pedicle (P) of the vertebra (V) so as to give a distal end portion (T1) of the hole a cylinder shape, and
- a milling cutter (13) designed to cut the bone material from the pedicle of the vertebra (V) so as to give a proximal end portion (T2) of the hole (T) a funnel shape gradually widening from the distal end portion (T1) of the hole (T), and
- a pedicle screw (20) that extends along a proximo-distal screw axis (X20),
**characterized in that**:
- the distal end (11) is pointed and centered on the drill axis (X1) for enabling driving into a bone material to be drilled, belonging to the pedicle (P) of the vertebra (V),
- the pedicle screw (20) is designed to be screwed into the hole, by being rotated on itself about the screw axis, after the drill bit (10) has drilled the hole (T) in the pedicle (P) of the vertebra (V) and has been removed from this hole, wherein the pedicle screw includes a threaded rod (21), which comprises a distal end (23) constituting a distal end of the pedicle screw (20), and which has a cylindrical shape, which is centered on the screw axis (X20) and whose nominal diameter (D21) is greater than a diameter (D12) of the drill (12) wherein the diameter (D12) of the drill bit (12) is substantially equal to the diameter (d21) of the threaded rod (21), measured at the base of the thread (22) of the threaded rod.

2. The system according to claim 1, wherein the milling cutter (13) has a conical shape that is centered on the drill axis (X10) and diverges proximally from the drill (12).

3. The system according to any of claims 1 or 2, wherein the drill (12) is provided with helical grooves with a cutting edge, which wind around the drill axis (X10) along the drill.

4. The system according to any one of the preceding claims, wherein the distal end (23) of the threaded rod (21) has a substantially conical shape, which is centered on the screw axis (X20) and diverges in the proximal direction.

5. The system according to any one of the preceding claims, wherein the system (1) further comprises an intraoperative control member (70), which is adapted to be introduced into the hole, after the drill bit (10) has drilled the hole (T) in the pedicle (P) of the vertebra (V) and has been removed from this hole and before the pedicle screw (20) is screwed into the hole, so as to control the positioning of the hole in the vertebra, in particular by palpation and/or radiography.

6. The system according to any one of the preceding claims, wherein the system (1) further comprises:
- a motorized device (30) adapted to rotate the drill bit (10) about the drill axis (X10) and/or to rotate the pedicle screw (20) about the screw axis (X20), and
- a robot (40) adapted to spatially move the motorized device (30).

7. The system according to claim 6, wherein the motorized device (30) is equipped with a force sensor (60) configured to measure at least one component of the forces exerted on the vertebra (V) by the drill bit (10) when drilling the hole (T) and/or by the pedicle screw (20) when screwing into the hole, and wherein the system (1) further comprises an electronic unit (50) adapted to control the movement of the motorized device (30) by the robot (40) as a function of said at least one component measured by the force sensor (60).

8. The system according to claim 7, wherein the electronic unit (50) is configured both to analyze said at least one component measured by the force sensor (60), in particular by comparing said at least one component to a predetermined value or by time tracking said at least one component, and to control the movement of the motorized device (30) by the robot (40) from the result of the analysis of said at least one component.

9. The system according to any of claims 7 or 8, wherein the electronic unit (50) is configured to control the movement of the motorized device (30) by the robot (40) so as to keep substantially constant the at least one component measured by the force sensor (60).
